# EUROPEAN PATENT SPECIFICATION

(11) **EP 2 286 745 B1**
(45) Date of publication and mention of the grant of the patent: **14.12.2016**
(21) Application number: 10172763.4
(22) Date of filing: 13.08.2010
(51) Int. Cl.: A61B 17/3203, B05B 17/06, A61B 17/00

(54) **FLUID EJECTION DEVICE**
Flüssigkeitsausgabevorrichtung
Dispositif d'éjection de fluide

(30) Priority: 17.08.2009 JP 2009188296
(43) Date of publication of application: 23.02.2011
(60) Divisional application: 16196536.3
(73) Proprietor: Seiko Epson Corporation, Tokyo 163-0811 (JP)
(72) Inventor: Kojima, Hideki, Nagano 392-8502 (JP); Sugimura, Shigeo, Nagano 392-8502 (JP); Ono, Yasuhiro, Nagano 392-8502 (JP)
(74) Representative: Hoffmann Eitle

(56) References cited:
- EP-A1- 2 039 515
- EP-A1- 2 283 785
- EP-A2- 2 022 419
- EP-A2- 2 078 504
- US-A1- 2005 147 502

## Description

### BACKGROUND

### 1. Technical Field

The present invention relates to a fluid ejection device for ejecting fluid in a pulsed manner.

### 2. Related Art

Document EP 2 022 419 A2 discloses a fluid injection device according to the preamble of claim 1.

Document EP 2 078 504 A2 discloses a fluid ejecting apparatus including: a fluid ejecting unit that ejects fluid from a fluid ejecting opening in pulses by reducing the capacity of a fluid chamber by a diaphragm; an inlet channel that supplies the fluid to the fluid chamber; and a check valve formed on the diaphragm and disposed between the inlet channel and the fluid chamber, in which the check valve closes the inlet channel when the capacity of the fluid chamber is reduced, and the check valve is bent when the capacity of the fluid chamber is expanded from the reduced state to open the inlet channel.

There are surgical instruments (fluid ejection devices) configured to incise or excise a living tissue by ejecting fluid at a high speed in a pulsed manner. The fluid ejection device includes a pulsed flow generating unit configured to transform fluid into a pulsed flow. The fluid ejection device is configured to eject the fluid at a high speed in the pulsed manner by driving the pulsed flow generating unit.

The fluid ejection device includes a one-input multi-control parameter changing unit configured to change a plurality of control parameters simultaneously. Fluid ejection conditions depend on the plurality of control parameters. The fluid ejection device is capable of ejecting fluid under adequate fluid ejection conditions by means of the one-input multi-control parameter changing unit (for example, United States Unexamined Patent Application No. 2009/0043480).

Fluid ejection conditions which are important in the case where the fluid ej ection device incises or excises a living tissue by ejecting fluid in a pulsed manner are an excision power per pulse and an excision speed per unit time. In the case of the fluid ejection device disclosed in United States Unexamined Patent Application No. 2009/0043480, the fluid ejection conditions are determined by selecting a set of fluid ejection conditions from a plurality of control parameters provided in advance.

In order for a user of the fluid ejection device to set detailed fluid ejection conditions, a huge number of combinations of parameters are necessary. However, it is difficult for the user to select optimal fluid ejection conditions on a case-by-case basis from among the huge number of combinations of the parameters.

### SUMMARY

The object of the invention is achieved by the subject-matter of the independent claim. Advantageous embodiments are disclosed in the dependent claims.

An advantage of some aspects of the invention is to solve at least part of the above-described problems. The invention can be implemented in the form of the following embodiment.

A fluid ej ection device includes: a fluid supplying unit configured to supply fluid at a predetermined fluid supply flow rate to a pressure chamber; a pulsed flow generating unit configured to generate a pulsed flow by varying the volume of the pressure chamber at a predetermined frequency and eject the pulsed flow from the pressure chamber; and a controller configured to control the fluid supplying unit and the pulsed flow generating unit so that the fluid supply flow rate is proportional to the frequency.

### BRIEF DESCRIPTION OF THE DRAWINGS

The disclosure will be described with reference to the accompanying drawings, wherein like numbers reference like elements.
Fig. 1 is a configuration drawing showing a fluid ejection device as a surgical instrument.
Fig. 2 is a cross-sectional view of a pulsed flow generator taken along the direction of ejection of fluid.
Fig. 3 is an explanatory block diagram showing a schematic configuration of a drive control unit.
Fig. 4 is a graph showing an example of a drive waveform of a piezoelectric element.
Fig. 5A is a schematic view showing volume variations in a pressure chamber in a state in which no voltage is applied to the piezoelectric element.
Fig. 5B is a schematic view showing the volume variations in the pressure chamber in a state in which voltage is applied on the piezoelectric element.
Fig. 6 is a graph schematically plotting the drive frequency versus the fluid supply flow rate.
Fig. 7 is a graph schematically showing drive waveforms of the piezoelectric element when the displacement volume is varied.
Fig. 8 is a graph schematically plotting the drive frequency versus the fluid supply flow rate when the displacement volume is varied.
Fig. 9 is a graph schematically showing an alternative drive waveform.
Fig. 10 is a graph schematically plotting the fluid supply flow rate versus the displacement volume.
Fig. 11 is a graph schematically plotting the displacement volume versus the fluid supply flow rate when the drive frequency is varied.
Fig. 12 is a graph schematically showing a drive waveform when a repetition frequency is lowered.
Fig. 13 is a graph schematically showing a drive waveform when the repetition frequency is increased.
Fig. 14 is a graph schematically showing a drive waveform when the repetition frequency is further increased.
Fig. 15 is a graph schematically plotting the product of the displacement volume and the drive frequency versus the fluid supply flow rate.

### DESCRIPTION OF EXEMPLARY EMBODIMENTS

Referring now to the drawings, embodiments of the disclosure will be described. A fluid ejection device according to the invention is employable for various application examples such as drawing using ink or the like, washing of fine substances or structures, and surgical knives. In the embodiments, the fluid ejection device suitable for incising or excising a living tissue will be described. Fluid using in the embodiments is liquid such as water or physiologic saline.

### First Embodiment

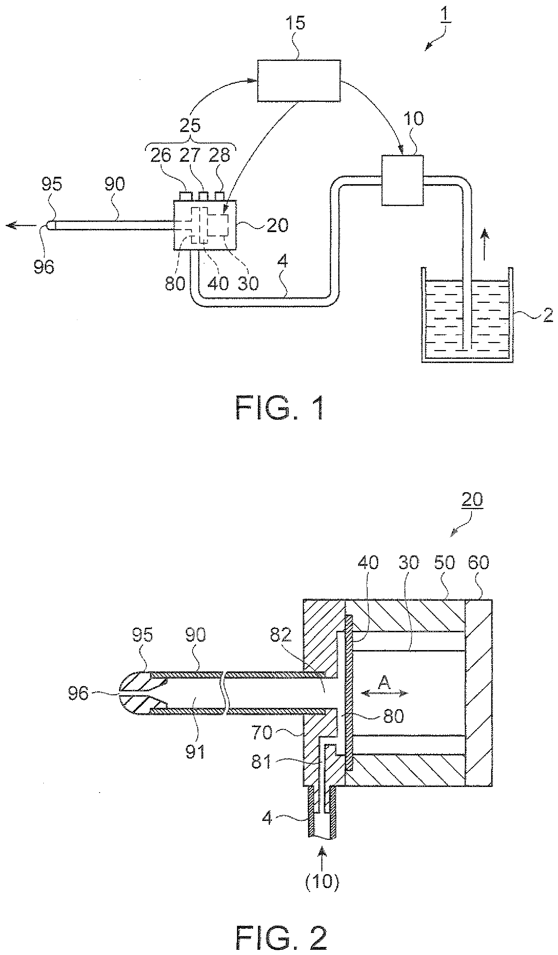

Fig. 1 is a configuration drawing showing the fluid ejection device as a surgical instrument. In Fig. 1, a fluid ejection device 1 includes a fluid supply container 2 in which fluid is stored, a pump 10 as a fluid supplying unit, a pulsed flow generator 20 as a pulsed flow generating unit configured to transform fluid supplied from the pump 10 into a pulsed flow, and a drive control unit 15 as a controller configured to control drive of the pump 10 and the pulsed flow generator 20. The pump 10 and the pulsed flow generator 20 are connected by a fluid supply tube 4.

A connecting flow channel tube 90 having a form of a thin pipe is connected to the pulsed flow generator 20. A nozzle 95 having a fluid ejection opening 96 with a reduced flow channel diameter is fixedly inserted to a distal end of the connecting flow channel tube 90.

The pulsed flow generator 20 includes a fluid ejection condition switching unit 25. The fluid ejection condition switching unit includes an excision power dial 26, an excision speed dial 27, and an ON/OFF switch 28.

The flow of fluid in the fluid ejection device 1 will be described. The fluid stored in the fluid supply container 2 is sucked by the pump 10 and is supplied to the pulsed flow generator 20 via the fluid supply tube 4 at a constant pressure. The pulsed flow generator 20 is provided with a pressure chamber 80 (see Fig. 2), and a piezoelectric element 30 and a diaphragm 40 as volume varying units configured to vary the volume of the pressure chamber 80. The pulsed flow generator 20 drives the piezoelectric element 30 to generate a pulsed flow in the pressure chamber 80, and ejects the fluid at a high speed in the pulsed manner via the connecting flow channel tube 90 and the nozzle 95.

The pulsed flow means fluid flow flowing in the constant direction and being associated with regular or irregular variations in flow rate or flow velocity of the fluid. The pulsed flow includes an intermittent flow in which flow and stop of the fluid are repeated, but does not necessarily have to be the intermittent flow.

Ejecting the fluid in a pulsed manner means ejection of fluid being associated with regular or irregular variations in flow rate or moving velocity of the ejected fluid. As an example of the ejection in the pulsed manner, there is an intermittent ejection in which ejection and non-ejection of the fluid are repeated. However, it does not necessarily have to be the intermittent ejection.

The structure of the pulsed flow generator 20 will be described. Fig. 2 is a cross-sectional view of the pulsed flow generator 20 according to the first embodiment taken along the direction of ejection of the fluid. The pulsed flow generator 20 includes an inlet flow channel 81 for supplying the fluid from the pump 10 into the pressure chamber 80 via the fluid supply tube 4, the piezoelectric element 30 and the diaphragm 40 as the volume varying units for varying the volume in the pressure chamber 80, and an outlet flow channel 82 being in communication with the pressure chamber 80. The fluid supply tube 4 is connected to the inlet flow channel 81.

The diaphragm 40 is formed of a disk-shaped metallic thin plate. The diaphragm 40 is in tight contact between a case 50 and a case 70. The piezoelectric element 30 is a stacked piezoelectric element. One of the both ends of the stacked piezoelectric element is secured to the diaphragm 40, and the other end is secured to a bottom plate 60.

The pressure chamber 80 is a space defined by a depression formed on a surface of the case 70 opposing the diaphragm 40 and the diaphragm 40. The pressure chamber 80 includes the outlet flow channel 82 opened at a substantially center portion thereof.

The case 70 and the case 50 are integrally joined at respective surfaces opposing to each other. The connecting flow channel tube 90 having a connecting flow channel 91 which communicates with the outlet flow channel 82 is fixedly fitted to the case 70, and the nozzle 95 is fixedly inserted to the distal end of the connecting flow channel tube 90. The nozzle 95 includes the fluid ejection opening 96 with a reduced flow channel diameter opened therethrough.

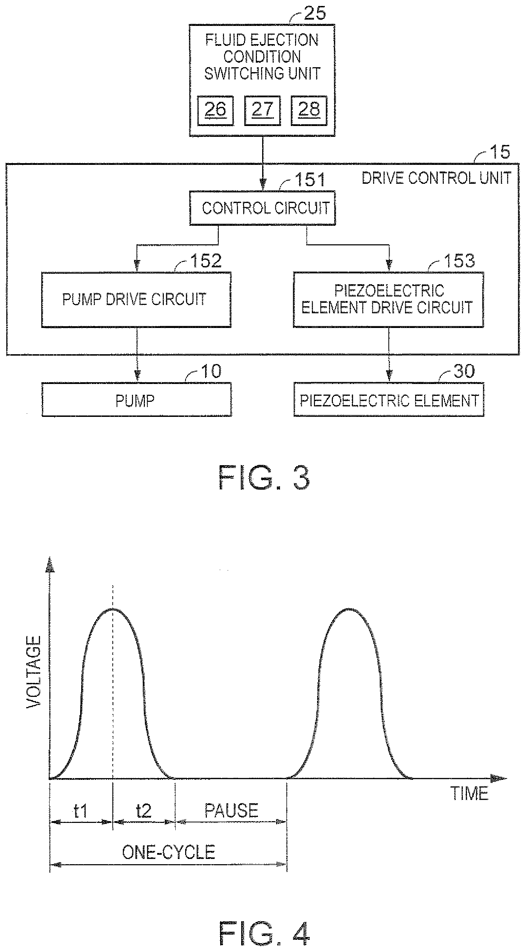

A configuration of a drive control unit will be described. Fig. 3 is a block diagram showing a schematic configuration of the drive control unit. The drive control unit 15 includes a pump drive circuit 152 configured to control drive of the pump 10, a piezoelectric element drive circuit 153 configured to control drive of the piezoelectric element 30, and a control circuit 151 configured to control the pump drive circuit 152 and the piezoelectric element drive circuit 153.

The control circuit 151 includes an arithmetic circuit (not shown) configured to calculate the drive frequency of the pump 10 which determines the fluid supply flow rate from the pump 10, the volume variations of the pressure chamber 80 which determines an incision power per pulse (the displacement volume discharged from the pressure chamber 80), and the frequency of the volume variations of the pressure chamber 80 which determines the excision velocity (which corresponds to the drive frequency of the piezoelectric element 30) on the basis of instructions from the excision power dial 26 and the excision speed dial 27. The piezoelectric element drive circuit 153 includes a waveform generation circuit configured to generate a predetermined drive waveform of the piezoelectric element 30 (not shown).

Referring now to Fig. 1 and Fig. 2, a fluid discharging action of the pulsed flow generator 20 will be described. A fluid discharge of the pulsed flow generator 20 is performed on the basis of the difference between a composite inertance L1 on the side of the inlet flow channel 81 and a composite inertance L2 on the side of the outlet flow channel 82.

The inertance will be described. An inertance L is expressed by L = ρ × h/S, where ρ is the density of the fluid, S is the cross-sectional area of the flow channel, and h is the length of the flow channel. A relation; ΔP = L × dQ/dt is derived by transforming a dynamic equation in the flow channel using the inertance L, where ΔP is the pressure difference in the flow channel, and Q is the flow rate of the fluid flowing in the flow channel.

The inertance L indicates the degree of influence affected on variations of flow rate with time. The larger the value of the inertance L, the smaller the variations of flow rate with time becomes. The smaller the value of the inertance L, the larger the variations of flow rate with time becomes.

The composite inertance L1 on the side of the inlet flow channel 81 is calculated within a range of the inlet flow channel 81. Since the fluid supply tube 4 which connects the pump 10 and the inlet flow channel 81 has flexibility, it may be excluded from the calculation of the composite inertance L1.

The composite inertance L2 on the side of the outlet flow channel 82 is an inertance within a range of the outlet flow channel 82 and the connecting flow channel 91. The thickness of a tube wall of the connecting flow channel tube 90 provides sufficient rigidity with respect to pressure propagation of the fluid.

The length and the cross-sectional area of the inlet flow channel 81 and the length and the cross-sectional area of the outlet flow channel 82 are designed so that the composite inertance L1 on the side of the inlet flow channel 81 becomes larger than the inertance L2 on the side of the outlet flow channel 82.

The fluid discharging action will be described. The fluid is supplied to the inlet flow channel 81 at a predetermined pressure by the pump 10. When the piezoelectric element 30 does not take any action, the fluid is allowed to flow into the pressure chamber 80 because of the difference between a discharge force of the pump 10 and a flow channel resistance of the entire part of the inlet flow channel 81.

When a drive signal is input to the piezoelectric element 30 and hence the piezoelectric element 30 is abruptly expanded in the direction vertical to a surface of the diaphragm 40 on the side of the pressure chamber 80 (direction of an arrow A), the diaphragm 40 is pressed by the expanded piezoelectric element 30. The diaphragm 40 is deformed in the direction to reduce the volume of the pressure chamber 80. If the composite inertances L1 and L2 on the side of the inlet flow channel 81 and on the side of the outlet flow channel 82 have enough magnitude, the pressure in the pressure chamber 80 rapidly rises to several tens of atmospheric pressure.

The pressure in the pressure chamber 80 is far higher than the pressure applied to the inlet flow channel 81 by the pump 10. An inflow of the fluid from the inlet flow channel 81 into the pressure chamber 80 is reduced by the pressure in the pressure chamber 80. An outflow of the fluid from the pressure chamber 80 to the outlet flow channel 82 is increased.

Since the composite inertance L1 on the side of the inlet flow channel 81 is larger than the composite inertance L2 on the side of the outlet flow channel 82, the amount of increase in fluid to be ejected from the outlet flow channel 82 is larger than the amount of decrease in flow rate flowing from the inlet flow channel 81 into the pressure chamber 80. Consequently, the pulsed fluid ejection (pulsed flow) occurs in the connecting flow channel 91. Pressure variations at the time of this ejection propagate in the interior of the connecting flow channel tube 90 (the connecting flow channel 91), and the fluid is ejected from the fluid ejection opening 96 of the nozzle 95 at the distal end.

As the flow channel diameter of the fluid ejection opening 96 is reduced from the flow channel diameter of the outlet flow channel 82, the fluid is subjected to a higher pressure, and hence is ejected at a high speed in the formed of pulsed liquid droplets.

The interior of the pressure chamber 80 is brought into a vacuum state immediately after the pressure rise because of a mutual action between reduction in amount of the inflow of the fluid from the inlet flow channel 81 and increase in amount of the outflow of the fluid from the outlet flow channel 82. When the piezoelectric element 30 is restored to its original shape, the fluid in the inlet flow channel 81 proceeds to the interior of the pressure chamber 80 at the same speed as that before action (before expansion) of the piezoelectric element 30 after elapse of a certain time because of both the pressure of the pump 10 and the vacuum state in the pressure chamber 80.

If the piezoelectric element 30 is expanded again after the flow of the fluid in the inlet flow channel 81 is restored, the pulsed liquid droplets are ejected continuously from the fluid ejection opening 96.

### Method of Driving Pulsed Flow Generator

A method of driving the pulsed flow generator 20 will be described. The drive waveform of the piezoelectric element 30 will be described. Fig. 4 is a graph showing an example of the drive waveform of the piezoelectric element. One cycle of the drive waveform corresponds to a time combining a sin waveform shifted by -90° in phase by being offset in the direction of a positive voltage and a pause. Assuming that the piezoelectric element 30 is expanded when the positive voltage is applied (direction indicated by the arrow A in Fig. 2), the section of a time t1 (hereinafter, referred to as voltage rise time t1) corresponds to the time during which the volume in the pressure chamber 80 is reduced. In the section of a time t2 (hereinafter, referred to as voltage drop time t2) is a section for charge elimination of the piezoelectric element 30 and, during this section, the piezoelectric element 30 is contracted. In the section of voltage drop time t2, the volume of the pressure chamber 80 increases.

A change of the frequency of the drive waveform is achieved by changing the length of the pause but not changing the voltage rise time t1. In other words, the through rate of voltage rise does not change. Therefore, the excision power per pulse is kept unchanged. The frequency of the volume variations of the pressure chamber 80 corresponds to the drive frequency of the piezoelectric element 30.

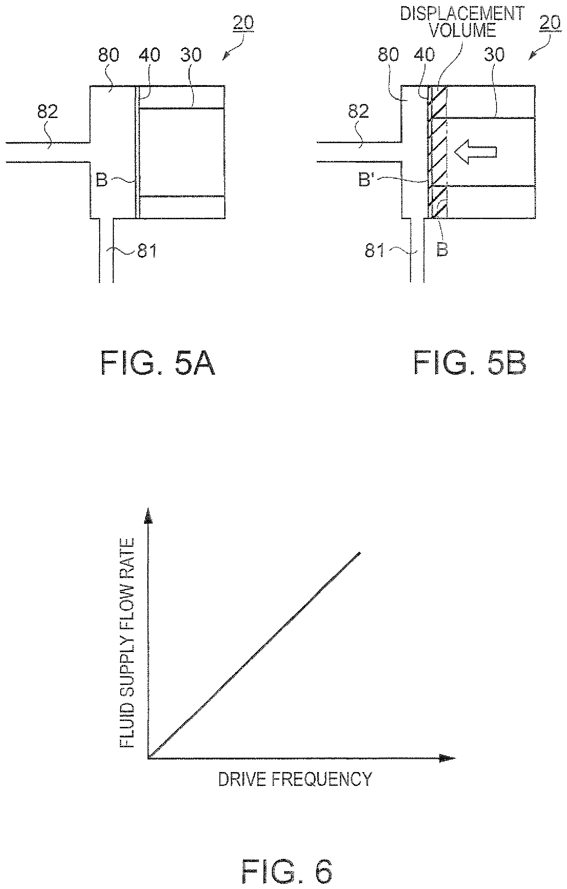

The volume variations in a pressure chamber in one cycle of the drive waveform will be described. Fig. 5A is a schematic view showing the volume variations in the pressure chamber in a state in which no voltage is applied on the piezoelectric element, and Fig. 5B is a schematic view showing the volume variations in the pressure chamber in a state in which the voltage is applied on the piezoelectric element. The volume variations during a voltage application time depend on the piezoelectric characteristic of the piezoelectric element 30. In the first embodiment, a case where the volume is reduced by applying voltage will be described for example. In Fig. 5A, the piezoelectric element 30 is in the state of not being applied with voltage. The volume of the pressure chamber 80 is also in the state of not being reduced (the position of the diaphragm 40 is shown by a line B).

The volume variations are expressed by the product of the displaceable surface area of the diaphragm 40 and the length of the elongation of the piezoelectric element 30. When a predetermined voltage is applied to the piezoelectric element 30, the volume of the pressure chamber 80 is reduced (the position of the diaphragm 40 is shown by a line B' in Fig. 5B) . When the diaphragm 40 is moved from B to B', the volume of the pressure chamber 80 varies by an amount indicated by a hatching in the drawing. The quantity of fluid corresponding to the volume variations is delivered from the outlet flow channel 82. The quantity of volume variations of the pressure chamber 80 is referred to as displacement volume of fluid.

If the gain of the drive voltage of the piezoelectric element 30 is fixed, the displacement volume caused by the piezoelectric element 30 is substantially constant. If the drive frequency of the piezoelectric element 30 is increased in the state in which the displacement volume is maintained constant, the fluid ejection flow rate is increased in proportion to the drive frequency. Therefore, the fluid supply flow rate from the pump 10 is needed to be increased in accordance with the fluid ejection flow rate.

### Fluid Ejection Method

The fluid ejection method will be described. Fig. 6 is a graph schematically plotting the drive frequency versus the fluid supply flow rate. A case where the drive frequency of the piezoelectric element 30 is varied when the displacement volume is in the default setting will be described. The excision power is set using the excision power dial 26. A required displacement volume is selected using the excision power dial 26, and then is fixed from then on. The setting of the excision power using the excision power dial 26 may be performed using the gain of the drive voltage, which is a condition to determine the displacement volume, may be used instead of the displacement volume if the condition such as the piezoelectric constant of the piezoelectric element 30 is already known.

The required drive frequency is selected by operating the excision speed dial 27 to set the fluid ejection flow rate. The fluid ejection flow rate is calculated by the product of the displacement volume and the drive frequency of the piezoelectric element 30. If the displacement volume is constant, the fluid ejection flow rate is increased in proportion to the drive frequency by increasing the drive frequency of the piezoelectric element 30. The excision speed is increased with the fluid ejection flow rate.

In order to increase the fluid ejection flow rate by increasing the drive frequency, it is necessary to increase the fluid supply flow rate from the pump 10. As shown in Fig. 6, the fluid supply flow rate from the pump 10 is determined to be proportional to the drive frequency of the piezoelectric element 30. The drive frequency of the piezoelectric element 30 and the fluid supply flow rate of the pump 10 are calculated by a calculator included in the control circuit 151. The drive frequency of the piezoelectric element 30 is input to the piezoelectric element drive circuit 153 and the fluid supply flow rate of the pump 10 is input to the pump drive circuit 152, whereby the piezoelectric element 30 and the pump 10 are driven under the respective drive conditions.

According to the first embodiment, if the frequency of the volume variations of the pressure chamber 80 (the drive frequency of the piezoelectric element 30) is increased, the fluid ejection flow rate is increased correspondingly. The fluid supply flow rate from the pump 10 is varied in proportion to the variations of the fluid ejection flow rate in association with the variations in drive frequency while maintaining the displacement volume of the pressure chamber 80 optimal (constant). The fluid supply flow rate can be secured as required, and the excision power per pulse and the excision speed can be adjusted adequately and independently. Accordingly, a user is allowed to operate the fluid ejection device easily under optimal fluid ejection conditions without preparing a huge number of combinations of parameters.

The probability of flowing out of excessive fluid from the nozzle 95 when the fluid is not being ejected is reduced by suppressing the fluid supply flow rate from becoming excessive. Therefore, the probability of occurrence of the problem of visibility deterioration of the operative site is low.

Subsequently, a description of the case of varying the displacement volume when the fluid supply flow rate and the drive frequency are in a proportional relationship (see Fig. 6) will be given below. The displacement volume is set by operating the excision power dial 26. The displacement volume is calculated by the product of the length of expansion of the piezoelectric element 30 and the movable surface area of the diaphragm 40. The length of expansion is determined by controlling voltage to be applied to the piezoelectric element 30. The excision power per pulse is determined by the displacement volume.

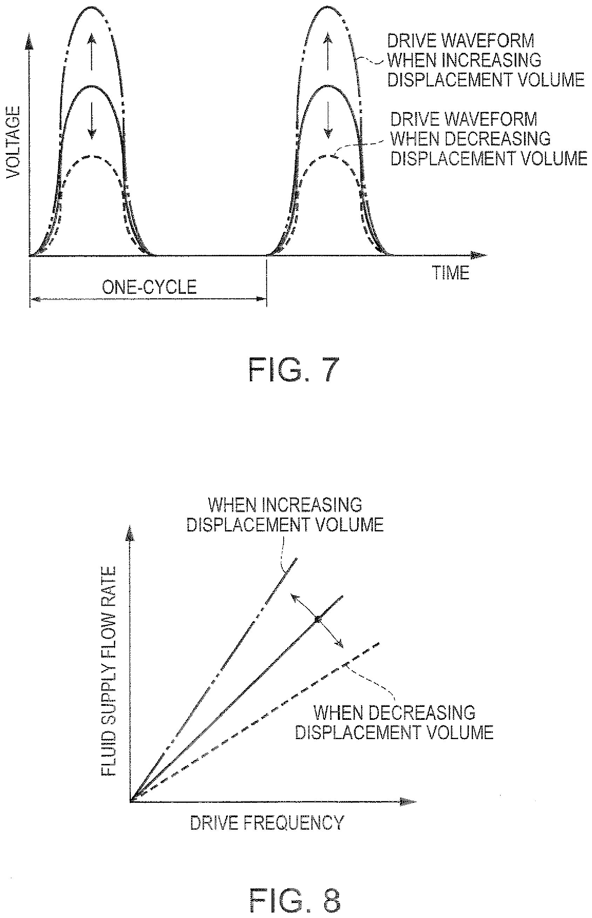

Fig. 7 is a graph schematically showing drive waveforms of the piezoelectric element when the displacement volume is varied. When adjusting the excision power per pulse with the excision power dial 26, an increase of the displacement volume is achieved by increasing the gain (voltage) of the drive waveform, and a reduction of the displacement volume is achieved by reducing the gain (voltage) of the drive waveform. The displacement volume is relatively varied by an amount corresponding to the gain of the drive waveform.

The drive frequency of the piezoelectric element 30 is set by operating the excision speed dial 27. Fig. 8 is a graph schematically plotting the drive frequency versus the fluid supply flow rate when the displacement volume is varied. The fluid supply flow rate is in a proportional relationship with respect to the frequency of the drive waveform (drive frequency) and is expressed by a straight line (see Fig. 6). The fluid supply flow rate is at least the same as the fluid ejection flow rate. The fluid ejection flow rate is calculated by the product of the displacement volume and the drive frequency of the piezoelectric element 30. Therefore, as shown in Fig. 8, the increase of the displacement volume is achieved by steepening the gradient of the straight line according to the amount of the increase of the displacement volume. In contrast, the reduction of the displacement volume is achieved by flattening the gradient of the straight line according to the amount of decrease of the displacement volume.

If the gain of the drive waveform is varied, the through rate of the voltage rise time t1 of the drive waveform also varies as shown in Fig. 7. To be exact, the fluid ejection flow rate and the displacement volume are not in the proportional relationship. Therefore, a change of the gradient of the straight line is achieved by storing data on the gradient of the straight line in the control circuit 151 (see Fig. 3) as a lookup table, and reading out the stored data from the lookup table when operating the excision power dial 26.

If the fluid supply flow rate and the drive frequency of the piezoelectric element 30 are in the proportional relationship, the fluid ejection flow rate is varied by varying the displacement volume. Therefore, the fluid supply flow rate may result in excess or deficiency. The variations in fluid supply flow rate can be changed by changing the gradient of the straight line according to the variations in the displacement volume. The displacement volume, that is, the excision power per pulse can be changed while compensating the excess and deficiency of the fluid supply flow rate adequately. The excision power per pulse and the excision speed per unit time can be adjusted independently over a wider range. The user can easily set the optimal fluid ejection conditions.

### Second Embodiment

The fluid ejection method according to a second embodiment will be described. In the second embodiment, the fluid supply flow rate is varied in proportion to the displacement volume. In a description of the second embodiment, the same components as the first embodiment are designated by the same reference numerals and description thereof is omitted.

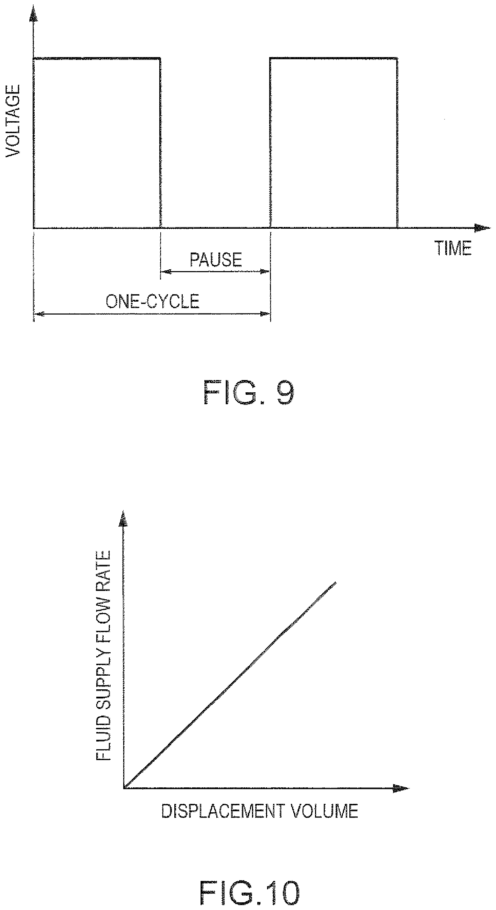

Fig. 9 is a graph schematically showing a drive waveform according to the second embodiment. Fig. 10 is a graph schematically plotting the fluid supply flow rate versus the displacement volume. The drive waveform illustrated in Fig. 9 is a rectangular wave. An increase of the frequency of the drive waveform is achieved by changing the length of the pause. Since the drive waveform is the rectangular wave, the through rate of the voltage rise does not change even though the gain of the drive voltage is changed. If the displacement volume per drive of the piezoelectric element is increased by increasing the gain of the drive voltage and increasing the displacement of the diaphragm 40, the fluid ejection flow rate is increased in proportion to the displacement volume.

The required displacement volume is selected by operating the excision power dial 26 while maintaining the drive frequency of the piezoelectric element 30 optimal (constant). On the basis of the selected displacement volume, a drive command is input from the control circuit 151 to the pump drive circuit 152 and the piezoelectric element drive circuit 153. Then, the pump 10 and the piezoelectric element 30 are driven, and then the fluid is supplied from the pump 10 at the fluid supply flow rate according to the variations in fluid ejection flow rate.

As the fluid ejection flow rate is proportional to the product of the displacement volume and the drive frequency, the fluid ejection flow rate varies in proportion to the variations in displacement volume. A supply of the fluid without excess and deficiency with respect to the fluid ejection flow rate is achieved only by varying the fluid supply flow rate in proportion to the variations in displacement volume as shown in Fig. 10.

In the second embodiment, the required fluid supply flow rate is secured by varying the fluid supply flow rate in proportion to the variations in displacement volume. The excision power per pulse and the excision speed per unit time can be adjusted independently. The optimal fluid ejection conditions can be set easily.

Suppression of excessive supply flow rate contributes to a reduction of the probability of flowing out of excessive fluid from the nozzle 95 when the fluid is not being ejected. Therefore, the probability of occurrence of the problem of visibility deterioration of the operative site is low.

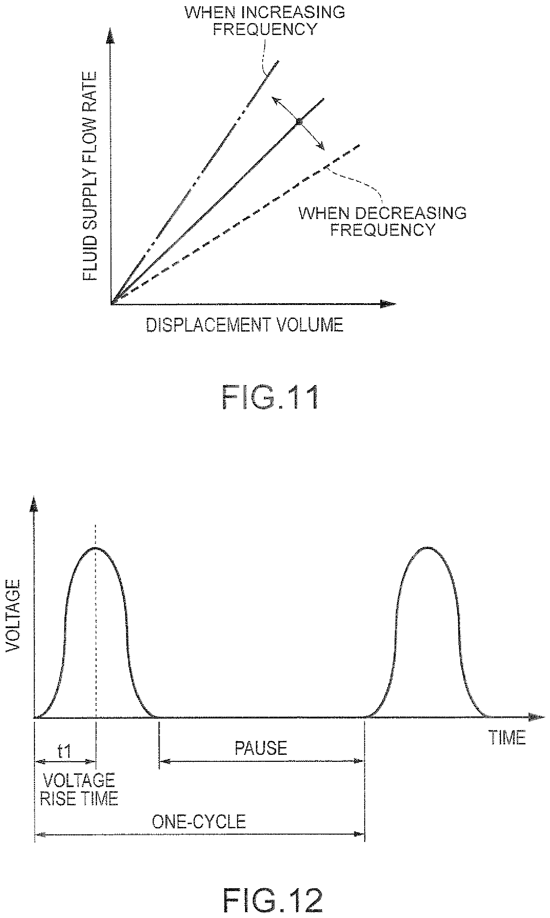

Subsequently, a description of the case of varying the drive frequency when the fluid supply flow rate and the displacement volume are in the proportional relationship (see Fig. 10) will be given below. Fig. 11 is a graph schematically plotting the displacement volume versus the fluid supply flow rate when the drive frequency is varied. The fluid supply flow rate is in the proportional relationship to the displacement volume, and is represented by a straight line with a certain gradient (see also Fig. 10).

The fluid ejection flow rate is calculated by the proportion of the product of the displacement volume and the drive frequency of the piezoelectric element 30. Therefore, an increase of the drive frequency is achieved by steepening the gradient of the straight line according to the amount of the increase of the drive frequency as shown in Fig. 11. In contrast, the reduction of the drive frequency is achieved by flattening the gradient of the straight line according to the amount of decrease of the drive frequency.

Depending on the drive waveform, there is a case where the through rate of the voltage rise of the drive waveform is varied with the variations in drive frequency. At this time, the fluid ejection flow rate is not proportional to the drive frequency to be exact. Therefore, the change of the gradient of the straight line is achieved by storing the data on the gradient of the straight line in the control circuit 151 (see Fig. 3) as the lookup table, and reading out the stored data from the lookup table when operating the excision speed dial 27.

The fluid ejection flow rate is varied by varying the drive frequency of the piezoelectric element 30. Therefore, the fluid supply flow rate may result in excess or deficiency. The variations in fluid supply flow rate can be changed by changing the gradient of the straight line according to the drive frequency of the piezoelectric element 30. The drive frequency of the piezoelectric element 30 (the excision speed per unit time) can be varied while compensating the excess or deficiency of the fluid supply flow rate adequately. The excision power per pulse and the excision speed per unit time can be adjusted independently over a wider range. The user can easily set the optimal fluid ejection conditions.

### Third Embodiment

The fluid ejection method according to a third embodiment will be described. In the third embodiment, the voltage rise time of the drive waveform of the piezoelectric element 30 with respect to the time during which the volume of the pressure chamber 80 is reduced is maintained substantially constant when varying the drive frequency. In a description of the third embodiment, the same components as the first embodiment are designated by the same reference numerals and description thereof is omitted.

A case where the repetition frequency is lowered when a drive waveform as that shown in Fig. 4 is employed as a basic drive waveform will be described. Fig. 12 is a graph schematically showing the drive waveform when a repetition frequency is lowered. In Fig. 12, the pause is elongated, and the voltage rise time t1 of the drive waveform of the piezoelectric element 30 is not changed with respect to the time during which the volume of the pressure chamber 80 is reduced. The through rate does not change either. Therefore, the drive frequency of the piezoelectric element 30 can be varied without changing the excision power per pulse. The excision power per pulse and the excision speed per unit time can be adjusted independently.

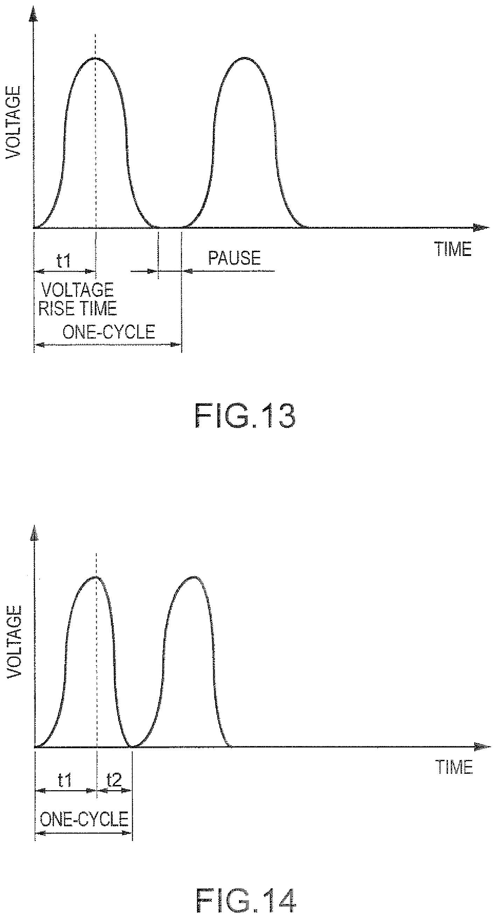

A case where the repetition frequency is increased will be described. Fig. 13 is a graph schematically showing a drive waveform when a repetition frequency is increased. In the drive waveform shown in Fig. 13, the pause is shorter with respect to the basic drive waveform (see Fig. 4), but still exists. The voltage rise time t1 does not change and the through rate does not change. Therefore, the drive frequency of the piezoelectric element 30 can be varied without changing the excision power per pulse.

A case where the repetition frequency is further increased will be described. Fig. 14 is a graph schematically showing a drive waveform when a repetition frequency is further increased. Fig. 14 shows a case where one cycle of the drive waveform is shorter than one cycle shown in Fig. 13. In the drive waveform shown in Fig. 14, no pause exists. The front and rear waveforms intersect if the intervals of the waveform shown in Fig. 13 are merely shortened. Therefore, a drive waveform in which the voltage drop time t2 enlarging the volume of the pressure chamber 80 is shorter than the basic waveform while the voltage rise time t1 does not change is employed. The voltage rise time t1 does not change and the through rate does not change. Therefore, the drive frequency of the piezoelectric element 30 can be varied without changing the excision power per pulse.

By maintaining the voltage rise time t1 constant, the through rate of the voltage rise time t1 is not varied even though the drive frequency is varied. Since the through rate is not varied, the excision power per pulse is kept unchanged. The excision speed can be varied while maintaining the excision power per pulse constant in comparison with the case of merely varying the drive frequency. The voltage rise time t1 must simply correspond to the time during which the volume of the pressure chamber 80 is reduced irrespective of the shape or polarity of the drive waveform of the piezoelectric element 30.

### Fourth Embodiment

The fluid ejection method according to a fourth embodiment will be described. In the fourth embodiment, the fluid supply flow rate from the pump 10 is set to be equal to the product of the displacement volume and the drive frequency, or to be larger than the product of the displacement volume and the drive frequency. In a description of the fourth embodiment, the same components as the first embodiment are designated by the same reference numerals and description thereof is omitted.

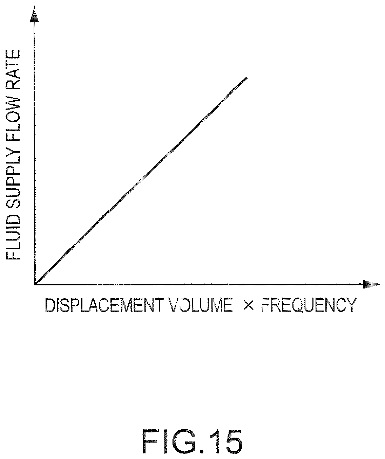

Fig. 15 is a graph schematically plotting the product of the displacement volume and the drive frequency versus the fluid supply flow rate. The fluid ejection flow rate is expressed by the product of the displacement volume and the drive frequency. Although the fluid supply flow rate and the fluid ejection flow rate are in a proportional relationship, if the fluid supply flow rate is smaller than the fluid ejection flow rate, the supply is insufficient, and hence the incision power per pulse is weakened. If the fluid supply flow rate is larger than the fluid ejection flow rate, the quantity of supply is excessive, and hence the fluid flows out from the nozzle 95 when the fluid is not being ejected, and the visibility of the operative site is lowered. Therefore, the required excision power per pulse is obtained and the favorable visibility is easily realized by equalizing the product of the volume variations and the drive frequency to the fluid ejection flow rate.

In the fluid ejection device configured to eject the fluid in the pulsed manner, the fluid may be drawn toward the fluid ejection opening 96 by the inertance effect of the fluid immediately after the fluid ejection, and hence may be flowed out by an amount larger than the displacement volume. As the fluid ejection flow rate becomes larger than the fluid supply flow rate, the excision power per pulse is weakened. Therefore, it is preferable to set the fluid supply flow rate to be larger than the product of the displacement volume and the drive frequency (fluid ejection flow rate) by at least an amount corresponding to an amount flowed out by the inertance effect.

If the fluid supply flow rate is set to be larger than the fluid ejection flow rate, excessive fluid may flow out from the nozzle 95 when the fluid is not being ejected, so that the visibility of the operative site may be deteriorated. It is preferable to set the fluid supply flow rate within a range not larger than double the product of the displacement volume and the drive frequency.

The amount of the fluid drawn toward the nozzle 95 by the inertance effect is smaller than the original displacement volume. If the fluid supply flow rate is set to be larger than the fluid ejection flow rate, it means that the stress is put on securing the excision power per pulse than preventing the excessive fluid from flowing out from the nozzle 95.

The required excision power per pulse is obtained and the favorable visibility is realized by equalizing the product of the displacement volume and the drive frequency to the fluid supply flow rate.

If the fluid supply flow rate is set to be larger than the fluid ejection flow rate, the required excision power per pulse is obtained and the influence on the visibility at the operative site may be reduced by setting the fluid supply flow rate to a value not larger than twice the product of the displacement volume and the drive frequency.

### Modification

In the embodiments described above, a configuration to generate the pulsed flow by displacing the diaphragm 40 by driving the piezoelectric element 30 is employed as the volume varying unit for the pressure chamber. It is also possible to employ a configuration to generate the pulsed flow by displacing a plunger (piston) by driving the piezoelectric element 30.

## Claims

1. A fluid ejection device (1) comprising:
a fluid supplying unit (10) configured to supply fluid at a predetermined fluid supply flow rate to a pressure chamber (80);
a pulsed flow generating unit (20) configured to generate a pulsed flow by varying the volume of the pressure chamber (80) at a predetermined frequency and eject the pulsed flow from the pressure chamber;
**characterized in that** the fluid ejection device further comprises
a controller (15) configured to control the fluid supplying unit (10) and the pulsed flow generating unit so that the fluid supply flow rate is proportional to the frequency.

2. The fluid ejection device according to claim 1, wherein the pulsed flow generating unit includes a piezoelectric element (30) varying the volume of the pressure chamber when voltage is applied to the piezoelectric element,
the controller (15) configured to control a voltage application time corresponding to the time during which the volume of the pressure chamber (80) is reduced to be maintained constant irrespective of the frequency.

3. The fluid ejection device according to claim 1 or 2, wherein the fluid supply flow rate is proportional to a displacement volume of the fluid discharged from the pressure chamber (80).

4. The fluid ejection device according to claim 3, wherein the fluid supply flow rate is not less than the product of the displacement volume and the frequency.

## Patentansprüche

1. Fluidausstoßvorrichtung (1) aufweisend:
eine Fluidversorgungseinheit (10), die eingerichtet ist, eine Druckkammer (80) mit Fluid bei einer vorbestimmten Fluidflussrate zu versorgen;
eine gepulste Flusserzeugungseinheit (20), die eingerichtet ist, einen gepulsten Fluss zu erzeugen, durch Variieren des Volumens der Druckkammer (80) bei einer vorbestimmten Frequenz, und den gepulsten Fluss aus der Druckkammer auszustoßen; **dadurch gekennzeichnet, dass** die Fluidausstoßvorrichtung weiter aufweist:
eine Steuereinheit (15), die eingerichtet ist, die Fluidversorgungseinheit (10) und die gepulste Flusserzeugungseinheit so zu steuern, dass die Fluidversorgungsflussrate proportional zu der Frequenz ist.

2. Die Fluidausstoßvorrichtung (1) gemäß Anspruch 1, wobei die gepulste Flusserzeugungseinheit ein piezoelektrisches Element (30) aufweist, welches das Volumen der Druckkammer variiert, wenn eine Spannung auf das piezoelektrische Element angewendet wird,
die Steuereinheit (15) eingerichtet ist, eine Spannungsanwendungszeit, die der Zeit entspricht, während welcher das Volumen der Druckkammer (80) verringert wird, so zu steuern, dass diese unabhängig von der Frequenz konstant bleibt.

3. Die Fluidausstoßvorrichtung (1) gemäß Anspruch 1 oder 2, wobei die Fluidversorgungsflussrate proportional zu einem Verschiebungsvolumen des Fluids ist, welches aus der Druckkammer (80) ausgestoßen wird.

4. Die Fluidausstoßvorrichtung (1) gemäß Anspruch 3, wobei die Fluidversorgungsflussrate nicht weniger als das Produkt des Verschiebungsvolumens und der Frequenz ist.

## Revendications

1. Dispositif d'éjection de fluide (1) comprenant :
une unité de fourniture de fluide (10) configurée pour fournir du fluide à un débit de fourniture de fluide prédéterminé à une chambre de pression (80) ;
une unité de génération de flux pulsé (20) configurée pour générer un flux pulsé en faisant varier le volume de la chambre de pression (80) à une fréquence prédéterminée et éjecter le flux pulsé de la chambre de pression ;**caractérisé en ce que** le dispositif d'éjection de fluide comprend en outre
un dispositif de commande (15) configuré pour commander l'unité de fourniture de fluide (10) et l'unité de génération de flux pulsé de sorte que le débit de fourniture de fluide soit proportionnel à la fréquence.

2. Dispositif d'éjection de fluide selon la revendication 1, dans lequel l'unité de génération de flux pulsé comporte un élément piézoélectrique (30) faisant varier le volume de la chambre de pression lorsqu'une tension est appliquée à l'élément piézoélectrique,
le dispositif de commande (15) configuré pour commander un temps d'application de tension correspondant au temps pendant lequel le volume de la chambre de pression (80) est réduit pour être maintenu constant quelle que soit la fréquence.

3. Dispositif d'éjection de fluide selon la revendication 1 ou 2, dans lequel le débit de fourniture de fluide est proportionnel à un volume de déplacement du fluide évacué de la chambre de pression (80).

4. Dispositif d'éjection de fluide selon la revendication 3, dans lequel le débit de fourniture de fluide n'est pas inférieur au produit du volume de déplacement et de la fréquence.
